# FASCICULE DE BREVET EUROPEEN

(11) **EP 1 196 450 B1**
(45) Date de publication et mention de la délivrance du brevet: **16.11.2005**
(21) Numéro de dépôt: 00949595.3
(22) Date de dépôt: 30.06.2000
(51) Int. Cl.: C07K 14/75, A61K 38/36, A61P 19/02, G01N 33/53

(54) **DERIVES CITRULLINES DE LA FIBRINE ET LEUR UTILISATION POUR LE DIAGNOSTIC OU LE TRAITEMENT DE LA POLYARTHRITE RHUMATO DE**
CITRULLIN-ENTHALTENDE FIBRIN-DERIVATE UND IHRE VERWENDUNG ZUR DIAGNOSE UND BEHANDLUNG VON RHEUMATISCHER ARTHRITIS
FIBRIN CITRULLINE DERIVATIVES AND THEIR USE FOR DIAGNOSING OR TREATING RHEUMATOID ARTHRITIS

(30) Priorité: 01.07.1999 FR 9908470
(43) Date de publication de la demande: 17.04.2002
(60) Demande divisionnaire: 05020523.6
(73) Titulaire: BIOMERIEUX SA, 69280 Marcy L'Etoile (FR)
(72) Inventeur: SERRE, Guy, Résidence du Lac, 31100 Toulouse (FR); SEBBAG, Mireille, F-31500 Toulouse (FR)
(74) Mandataire: Vialle-Presles, Marie José
(86) Numéro de dépôt international: PCT/FR2000/001857
(87) Numéro de publication internationale: WO 2001/002437

(56) Documents cités:
- WO-A1-95/28946
- WO-A2-98/22503

## Description

La présente invention est relative à des dérivés citrullinés de fibrine, et à leurs utilisations dans le diagnostic et le traitement de la polyarthrite rhumatoïde.

La polyarthrite rhumatoïde (ci après abrégée en " PR ") est le plus fréquent des rhumatismes inflammatoires chroniques. Il s'agit d'une maladie auto-immune ; le sérum des patients atteints contient des auto-anticorps dont certains sont spécifiques, et peuvent constituer un marqueur de cette maladie, permettant son diagnostic même à des stades précoces.

Des travaux antérieurs de l'équipe des Inventeurs ont montré que ces anticorps reconnaissaient différentes formes moléculaires de la famille des (pro)filaggrines (pour revue, cf. par exemple. SERRE et VINCENT, In : Autoantibodies, PETER and SHOENFELD Eds, Elsevier Science Publishers, 271-276, 1996). Ces anticorps ont pour cette raison été dénommés : « auto-anticorps anti-filaggrine (AAF) » ;. Les Demandes EP 0 511 116 et WO 9822503 décrivent la purification et la caractérisation d'antigènes de la famille des filaggrines reconnus par ces anticorps, et leur utilisation pour le diagnostic de la polyarthrite rhumatoïde.

Les Inventeurs ont montré que les épitopes reconnus par les AAF étaient portés par des régions de la molécule de filaggrine dans lesquelles au moins une partie des arginines étaient déiminées, et transformées de la sorte en citrulline ; des peptides citrullinés spécifiquement reconnus par les AAF ont ainsi été obtenus à partir des principales régions immunoréactives de la filaggrine. Ces peptides, et leur utilisation pour le diagnostic de la PR font l'objet de la Demande PCT/FR97/01541, et de la Demande PCT/FR98/02899 au nom de BIOMERIEUX. Les observations des Inventeurs concernant le rôle de résidus citrulline dans la réactivité de la filaggrine avec les auto-anticorps spécifiques de la PR ont été ultérieurement confirmées par d'autres chercheurs [SCHELLEKENS et al., Arthritis Rheum., 40, n° 9 supplément, p. S276, résumé 1471 (1997); VISSER et al., Arthritis Rheum., 40, n° 9 supplément, p. S289, résumé 1551 (1997)].

Les Inventeurs ont en outre montré que les AAF représentaient une proportion importante des immunoglobulines interstitielles des tissus rhumatoïdes synoviaux et qu'ils étaient synthétisés localement par des plasmocytes spécifiques présents dans ces tissus, ce qui confirme l'hypothèse de leur implication dans la réponse auto-immune associée à la PR. L'utilisation de la filaggrine ou de peptides citrullinés dérivés de celle-ci pour neutraliser cette réponse auto-immune fait l'objet de la Demande PCT/FR98/02900 au nom de l'UNIVERSITÉ PAUL SABATIER (TOULOUSE III).

Toutefois, l'implication de la filaggrine comme immunogène ou comme antigène-cible dans la réponse auto-immune associée à la PR n'a jamais été constatée. Le véritable antigène impliqué dans cette réponse restait à identifier.

Les Inventeurs sont maintenant parvenus à caractériser cet antigène, et ont ainsi montré qu'il se composait de dérivés citrullinés des chaînes α et/ou β de la fibrine.

La présente invention a pour objet un polypeptide citrulliné reconnu par des autoanticorps anti-filaggrine spécifiques de la polyarthrite rhumatoïde caractérisé en ce qu'il est dérivé de tout ou partie de la séquence de la chaîne α ou de la chaîne β d'une fibrine de vertébré, par substitution d'au moins un résidu arginine par un résidu citrulline.

De préférence, un polypeptide conforme à l'invention comprend au moins 5 acides aminés consécutifs, et avantageusement au moins 10 acides aminés consécutifs, dont au moins une citrulline, de la séquence de la chaîne α ou de la chaîne β d'une fibrine de mammifère. Avantageusement ladite fibrine de vertébré est une fibrine de mammifère, de préférence humaine.

Des polypeptides citrullinés conformes à l'invention peuvent par exemple être obtenus à partir de fibrine ou de fibrinogène naturels, recombinants, ou de synthèse, ou de fragments de ceux-ci comprenant au moins un résidu arginine, par l'action de la peptidyl arginine déiminase (PAD) ; ils peuvent également être obtenus par synthèse peptidique, en incorporant directement un ou plusieurs résidus citrulline, dans le peptide synthétisé.

Des polypeptides citrullinés conformes à l'invention peuvent également être des pseudopeptides, possédant la même structure tridimensionnelle, et donc la même réactivité immunologique que les polypeptides citrullinés dérivés des chaînes α ou β de fibrine ou de leurs fragments, mentionnés ci-dessus. Il peut s'agir par exemple de pseudopeptides de type rétro, dans lesquels des acides L-aminés sont enchaînés selon une séquence inverse de celle du peptide à reproduire, ou bien de pseudopeptides de type *rétro-inverso,* constitués par des acides aminés de la série D (au lieu des acides aminés de la série L des peptides naturels) enchaînés selon une séquence inverse de celle du peptide à reproduire, ou bien encore de pseudopeptides contenant une liaison CH₂-NH à la place d'une liaison peptidique CO-NH. Des pseudopeptides de ces différents types sont par exemple décrits par BENKIRANE et al. [J. Biol. Chem., 270, p. 11921-11926, (1995) ; J. Biol. Chem., 271, p. 33218-33224, (1996)] ; BRIAND et al. [J. Biol. Chem., 270, p. 20686-20691, (1995) ; GUICHARD et al. [J. Biol. Chem., 270, p. 26057-26059, (1995)].

La présente invention a également pour objet l'utilisation des polypeptides conformes à l'invention, tels que définis ci-dessus, pour le diagnostic *in vitro* de la PR.

La présente invention englobe en particulier des compositions antigéniques pour le diagnostic de la présence d'auto-anticorps spécifiques de la PR dans un échantillon biologique, lesquelles compositions sont caractérisées en ce qu'elles contiennent au moins un polypeptide conforme à l'invention, éventuellement marqué et/ou conjugué avec une molécule porteuse.

La présente invention a également pour objet un procédé de détection des auto-anticorps de classe G spécifiques de la PR dans un échantillon biologique, lequel procédé est caractérisé en ce qu'il comprend :
- la mise en contact dudit échantillon biologique avec au moins un polypeptide conforme à l'invention, tel que défini ci-dessus, dans des conditions permettant la formation d'un complexe antigène/anticorps avec les auto-anticorps spécifiques de la PR éventuellement présents ;
- la détection, par tous moyens appropriés, du complexe antigène/anticorps éventuellement formé.

Ce procédé de détection peut être mis en oeuvre grâce à un nécessaire comprenant au moins un antigène selon l'invention, ainsi que des tampons et réactifs appropriés pour la constitution d'un milieu réactionnel permettant la formation d'un complexe antigène/anticorps, et/ou des moyens de détection dudit complexe antigène/anticorps.

Ledit nécessaire peut également comprendre, le cas échéant, des échantillons de référence, tels qu'un ou plusieurs sérum(s) négatif(s) et un ou plusieurs sérum(s) positif(s).

La présente invention a aussi pour objet l'utilisation de polypeptides citrullinés conformes à l'invention pour l'obtention d'un médicament, et notamment d'un médicament destiné à neutraliser la réponse auto-immune associée à la PR, et en particulier à inhiber la fixation des effecteurs humoraux ou cellulaires de cette réponse auto-immune avec les dérivés citrullinés de chaînes α ou β de fibrine présents dans les tissus rhumatoïdes.

Cette neutralisation in vivo de la réponse auto-immune, peut participer au traitement de la PR, ou d'autres maladies dans lesquelles interviendraient des lésions induites par une réponse auto-immune dirigée contre des épitopes présentant des réactions croisées avec les dérivés citrullinés de chaînes α ou β de fibrine.

Avantageusement, pour l'administration *in vivo*, on choisira des polypeptides modifiés de manière à prolonger leur durée de vie dans l'organisme, en particulier en augmentant leur résistance aux protéases ; il peut s'agir en particulier de pseudopeptides, tels que ceux mentionnés ci-dessus.

La présente invention englobe également des compositions pharmaceutiques, notamment pour le traitement de la polyarthrite rhumatoïde, caractérisées en ce qu'elles contiennent en tant que principe actif, au moins un polypeptide conforme à l'invention.

Des compositions pharmaceutiques conformes à l'invention peuvent être administrées par tous moyens appropriés, connus en eux-mêmes. Elles peuvent par exemple être administrées de manière systémique, par voie orale, ou par voie parentérale, en injection sous-cutanée, intraveineuse ou intramusculaire ; elles peuvent également être administrées localement, par exemple par injections intra-articulaires, ou par micro-injections sous arthroscopie dans le tissu synovial inflammatoire.

La présente invention sera mieux comprise à l'aide du complément de description qui va suivre, qui se réfère à l'identification de formes déiminées de la chaîne α et de la chaîne β de la fibrine humaine dans les tissus rhumatoïdes, et à l'utilisation de fibrinogène déiminé pour la détection de la présence d'AAF dans des échantillons de sérum.

### EXEMPLE 1 : PURIFICATION ET CARACTERISATION DE PROTEINES ANTIGENIQUES RECONNUES PAR LES AAF DANS LES TISSUS SYNOVIAUX RHUMATOÏDES

### 1) Analyse des tissus synoviaux rhumatoïdes

### Matériel et méthodes

Les échantillons de tissu synovial utilisés pour les extractions protéiques ont été prélevés chez des patients atteints de polyarthrite rhumatoïde, au moment d'une synovectomie ou d'une arthroplastie du poignet ou du genou et correspondent tous à des fragments tissulaires qui sont le siège de lésions histologiques classiques de synovite rhumatoïde. Ils sont conservés par congélation dans de l'isopentane refroidi par de l'azote liquide.

Des fragments de tissu synovial provenant de quatre patients ont été extraits de manière séquentielle, successivement dans un tampon de faible force ionique, un tampon urée, puis un tampon urée/DTT.

### Préparation des extraits synoviaux

L'extraction a été effectuée à l'aide d'un homogénéiseur Ultra-Turrax (T25 basic, IKA Labortechnik, Staufen, Germany) avec un volume de 6 ml de tampon par gramme de tissu.

Les tampons suivants ont été utilisés à température de 0° C: Tris 40 mM- HCl, pH 7,4, contenant 150 mM de NaCl [tampon de faible force ionique]; Tris 40 mM- HCl, pH 7,4, contenant de l'urée 8M désionisée sur une résine échangeuse d'ions (AG 501-X8, Biorad, Hercules, CA) [tampon urée]; Tris 40 mM- HCl, pH 7,4, contenant de l'urée 8M désionisée et du dithiothréitol (DTT) 50 mM, (Sigma) [tampon urée/DTT]. Tous les tampons ont été supplémentés par de l'EDTA 20 mM, de l'azide de sodium à 0,02%, de l'aprotinine à 2 µg/ml, du Néthylmaléimide 10 mM et du phénylméthylsulfonyl fluoride 1 mM (Sigma, Saint Louis, MI). Après chaque extraction, les homogénats ont été centrifugés pendant 20 minutes à 15000 g, à la température de 4°C. Les extraits en tampon urée et en tampon urée/DTT ont été dialysés contre de l'eau avant d'être analysés par électrophorèse et par immunotransfert .

### Electrophorèse et immunodétection

Les protéines synoviales des différents extraits ont été séparées par électrophorèse en gel de polyacrylamide 10 % en tampon SDS dénaturant (PAGE-SDS) puis ont été électrotransferrées sur des membranes de nitrocellulose renforcée (Hybond-^{TM}C extra, Amersham, Little Chalfont, UK).

Les membranes ont été immunodétectées avec les préparations d'anticorps suivantes : sérums humains rhumatoïdes AAF-positifs ou AAF-négatifs ; sérums humains contrôles non-rhumatoïdes issus de patients atteints d'autres rhumatismes inflammatoires ou d'individus sains (1/100) ; fractions d'AAF purifiés (10 µg/ml) ; anticorps monoclonal de souris dirigé contre la fibrine et le fibrinogène humain (5 µg/ml) ; deux antisérums de mouton respectivement dirigés contre les chaînes α et γ recombinantes du fibrinogène humain (1/1000) (Cambio, Cambridge, UK) ; un antisérum de lapin dirigé contre la chaîne β recombinante du fibrinogène humain (1/200000) (Cambio).

Les sérums humains utilisés sont issus de 95 patients atteints de polyarthrite rhumatoïde (PR) parfaitement caractérisés sur les plans clinique et biologique selon les critères de l'American College of Rheumatology, de 24 patients atteints de rhumatismes inflammatoires non rhumatoïdes ou de pathologies non inflammatoires (sérums contrôles) et de 10 individus sains. La titration semi-quantitative des anticorps anti-filaggrine (AAF) dans les sérums a été réalisée par immunofluorescence indirecte sur cryocoupes d'épithélium d'oesophage de rat et par immunotransfert sur extraits épidermiques enrichis en variant acide de la filaggrine, selon des protocoles précédemment publiés [VINCENT et al., Ann. Rheum. Dis., 48, 712-722, (1989) ; VINCENT et al., J. Rheumatol., 25, 838-846, (1998)]. Les sérums dits «AAF-positifs» sont ceux qui présentent des AAF à des titres significatifs après détection par les deux méthodes, et les sérums dits «AAF-négatifs» sont ceux qui ne présentent d'AAF détectables par aucune des deux méthodes.

Les AAF ont été purifiés par chromatographie d'affinité sur le variant acide de la filaggrine épidermique selon le protocole décrit par GIRBAL-NEUHAUSER et al. (J. Immunol., 162, 585-594, (1999), à partir de 45 sérums rhumatoïdes de haut titre en AAF. Les fractions d'anticorps purifiés ont été réunies.

Des sondes moléculaires secondaires conjuguées à la peroxydase ont été utilisées pour la détection de tous les anticorps primaires: protein-A (Sigma), anticorps de mouton dirigés contre les IgG de souris, (Biosys, Compiègne, France), fragments Fab de chèvre dirigés contre les IgG de lapin (Biosys) et fragments F(ab')2 de lapin dirigés contre les IgG de mouton (Southern Biotech. Inc), pour la détection respective des IgG humaines, murines, de lapin, et de mouton. L'activité peroxydase a été visualisée par le système de détection ECL^{TM} (Amersham International, Aylesbury, UK), suivant le protocole proposé par le fabricant.

### Résultats

Une réactivité spécifique avec les AAF purifiés et les sérums rhumatoïdes AAF-positifs a été observée uniquement dans l'extrait réalisé en tampon urée/DTT.

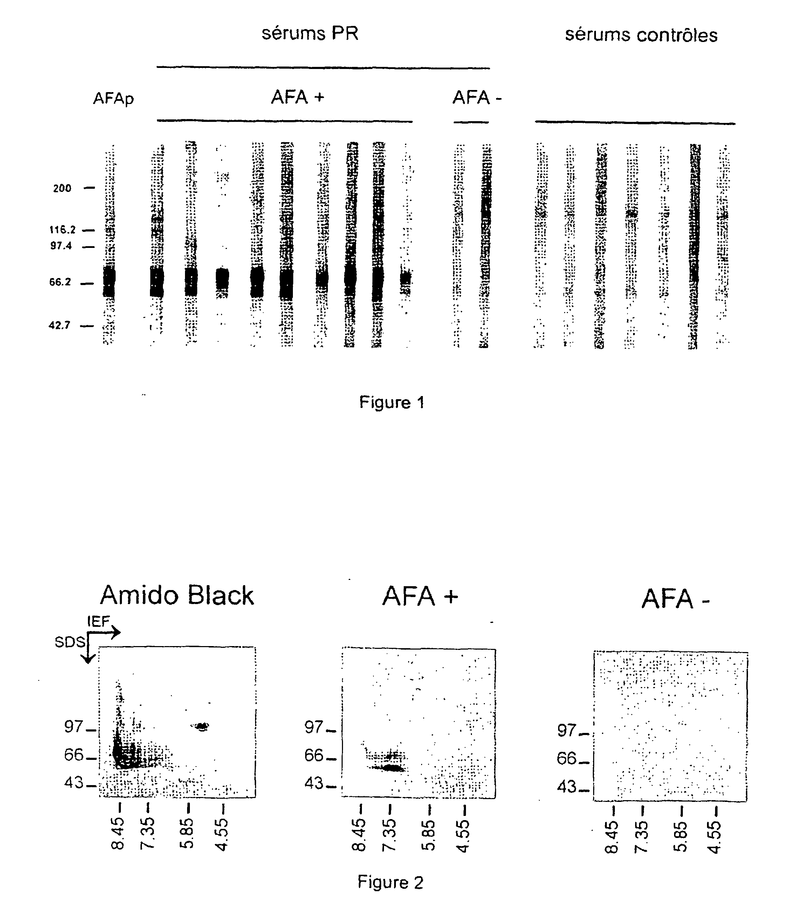

Les résultats sont illustrés par la Figure. 1.

### Légende de la Figure 1 :

- AFAp = AAF purifiés ;
- sérums PR = sérums rhumatoides :
   * AFA+ = AFA-positifs ;
   * AFA- = AFA-négatifs ;
- sérums contrôles =sérums issus de patients atteints d'autres rhumatismes inflammatoires que la PR ou de donneurs sains.

Ces résultats montrent que la réactivité spécifique avec les AAF purifiés et les sérums rhumatoïdes AAF-positifs concerne deux bandes protéiques de poids moléculaire apparent d'environ 64 kD à environ 78 kD (p64-78) et d'environ 55 kD à environ 61 kD (p55-61), respectivement. Ces bandes protéiques n'ont pas été détectées par les sérums AAF-négatifs, qu'ils proviennent de patients atteints de PR ou d'autres rhumatismes inflammatoires, ou bien qu'ils soient issus de donneurs sains.

La présence de ces protéines spécifiquement reconnues par les AAF purifiés et les sérums rhumatoïdes AAF-positifs, a été observée dans les extraits urée/DTT de tissus synoviaux issus des 4 patients rhumatoïdes étudiés.

Au total 48 sérums rhumatoïdes AAF-positifs ont été testés en immunotransfert sur au moins un extrait synovial urée/DTT. Parmi ces sérums, 40 ont reconnu p64-78, 39 ont reconnu p55-61, 37 ont reconnu à la fois p64-78 et p55-61, 3 n'ont reconnu que p64-78 et 2 n'ont reconnu que p55-61.

Treize sérums rhumatoïdes AAF-négatifs ont été testés en immunotransfert sur au moins un extrait urée/DTT de tissu synovial ; aucun de ces sérums n'a reconnu p64-78 ou p55-61.

Dix sérums issus de donneurs sains et 5 sérums issus de patients atteints d'autres rhumatismes inflammatoires ont aussi été testés en immunotransfert sur au moins un extrait synovial urée/DTT ; aucun de ces sérums n'a reconnu p64-78 ou p55-61.

### 2) Caractérisation des protéines antigéniques p64-78 et p55-61.

Les protéines de l'extrait en tampon urée/DTT du tissu synovial de l'un des patients atteint de PR, ont été précipitées par 4 volumes d'acétone glacial, puis redissoutes dans le tampon urée/DTT à une concentration 15 fois supérieure à leur concentration initiale.

Les protéines de l'extrait concentré ont été séparées en électrophorèse bidimensionnelle par isoélectrofocalisation suivie de SDS-PAGE.

Une séparation électrophorétique bidimensionnelle a été réalisée dans le système PhastSystem^{TM} (Pharmacia). La première séparation électrophorétique a eu lieu sur des gels d'isoélectrofocalisation (IEF) PhastGels^{TM} préalablement lavés, séchés et réhydratés dans un tampon désionisé contenant de l'urée 8 M, du Nonidet P-40 à 0,5% et des ampholytes créant un gradient de pH de 3 à 10 (Pharmacia). La deuxième dimension a été réalisée en SDS-PAGE sur des gels à 7,5% de polyacrylamide.

Les protéines ont ensuite été électrotransferrées sur des membranes de polyvinyldifluoride (PVDF) (membranes ProBlott^{TM} , Applied Biosystems, Foster City, CA), en Tris 50 mM et acide borique 50 mM. Les membranes ont enfin été colorées par une solution aqueuse d'amido black à 0,1 %, d'acide acétique à 1 % et de méthanol à 45 % , ou bien immunodétectées par des sérums rhumatoïdes selon le protocole décrit en 1) ci-dessus.

La Figure 2 illustre les profils obtenus après électrotransfert sur membrane de PVDF et :
a) coloration à l'amido-black ; ou bien
b) immunodétection par un sérum rhumatoïde AAF-positif ; ou bien
c) immunodétection par un sérum rhumatoïde AAF-négatif .

### Légende de la Figure 2 :

- Amido Black = coloration à l'amido-black ;
- AFA+ = immunodétection avec un sérum rhumatoïde AAF-positif ;
- AFA- = immunodétection avec un sérum rhumatoïde AAF-négatif.

Après coloration à l'amido-black, on observe la présence de deux protéines majoritaires, de poids moléculaire apparent 64-78 kD et 55-61 kD et de pI d'environ 5,85 à environ 8,45.

Ces protéines sont immunodétectées par les sérums rhumatoïdes AAF-positifs, mais pas par les sérums rhumatoïdes AAF-négatifs.

À partir de transferts identiques sur membrane de PVDF après électrophorèse bidimensionnelle, des fragments de membrane correspondant au centre de chaque zone immunoréactive ont été excisés puis soumis à un séquençage amino-terminal dans un séquenceur Applied Biosystems (494A ou 473A) selon le procédé conseillé par le fabricant.

À partir du fragment de membrane correspondant à l'antigène p64-78, la séquence gly-pro-arg-val-val-glu-arg-his-gln-ser-ala a été obtenue. Cette séquence est strictement identique à la séquence 36-46 du produit du gène du précurseur de la chaîne α du fibrinogène humain. Lorsque des fragments de membrane correspondant aux extrémités droite ou gauche de la zone immunoréactive p64-78 ont été excisés puis soumis chacun à trois cycles de séquençage amino-terminal, les séquences gly-pro-arg ont été retrouvées à chaque fois, indiquant que la totalité de la zone immunoréactive p64-78 possède la même extrémité amino-terminale.

À partir du fragment de membrane correspondant au centre de la zone immunoréactive correspondant à l'antigène p55-61, la séquence gly-his-arg-pro-leu-asp-lys-lys-arg a été obtenue. Cette séquence est strictement identique à la séquence 45-54 du produit du gène précurseur de la chaîne β du fibrinogène humain. Lorsqu'un fragment de membrane correspondant à l'extrémité gauche de la zone immunoréactive p55-61 a été excisé puis soumis à deux cycles de séquençage amino-terminal, la séquence gly-his a été retrouvée. Lorsqu'un fragment de membrane correspondant à l'extrémité droite de la zone immunoréactive p55-61 a été excisé puis soumis à six cycles de séquençage amino-terminal, la séquence gly-his-arg-pro-leu-asp et la séquence gly-pro-arg-val-val-glu ont été retrouvées. Ceci indique que la totalité de la zone immunoréactive p55-61 possède la même extrémité amino-terminale et qu'elle comigre partiellement avec l'antigène p64-78.

Les extrémités aminoterminales des protéines antigéniques p64-78 et p55-61 correspondent respectivement aux extrémités aminoterminales des chaînes α et β du fibrinogène humain après clivage respectif par la thrombine des fibrinopeptides A et B. Les extrémités aminoterminales des protéines antigéniques p64-78 et p55-61 sont donc respectivement identiques à celle de la chaîne α et à celle de la chaîne β de la fibrine humaine.

Les poids moléculaires apparents des antigènes p64-78 et p55-61 sont compatibles avec les valeurs respectives de poids moléculaires théoriques de la chaîne α et de la chaîne β de la fibrine humaine.

L'identité de l'antigène p64-78 et de la chaîne α de la fibrine d'une part, et celle de l'antigène p55-61 et de la chaîne β de la fibrine d'autre part, ont été confirmées par analyse de la réactivité d'anticorps antifibrin(ogèn)e vis-à-vis de ces antigènes. En immunotransfert, à partir d'un extrait de tissu synovial réalisé en urée/DTT, l'anticorps monoclonal de souris "311" qui reconnaît les trois chaînes α, β et, faiblement, γ du fibrinogène et de la fibrine humaine, est majoritairement réactif vis-à-vis des antigènes p64-78 et p55-61. De même, deux antisérums, l'un de mouton et l'autre de lapin, dirigés respectivement contre les chaînes α et β recombinantes du fibrinogène, ont principalement reconnu, respectivement, une protéine qui comigrait avec l'antigène p64-78 et une protéine qui comigrait avec l'antigène p55-61.

### EXEMPLE 2 : REACTIVITE DE SERUMS RHUMATOÏDES ET D'AAF PURIFIES AVEC DU FIBRINOGENE DEIMINE IN VITRO.

La réactivité vis-à-vis du fibrinogène déiminé et non déiminé a été étudiée par immunotransfert. Ont été utilisés : les fractions d'AAF purifiés, 37 sérums rhumatoïdes AAF-positifs de titre décroissant, 10 sérums rhumatoïdes AAF-négatifs et 19 sérums AAF-négatifs issus de patients atteints de rhumatismes inflammatoires ou non inflammatoires (titres en AAF déterminés par immunotransfert sur extraits épidermiques enrichis en variant acide de la filaggrine).

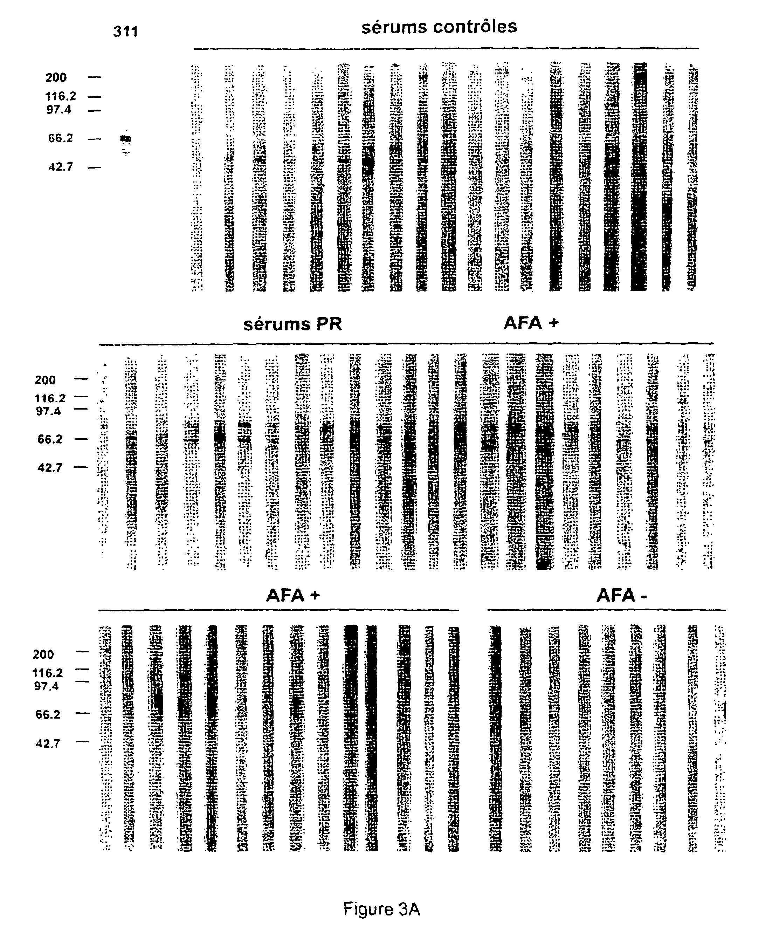

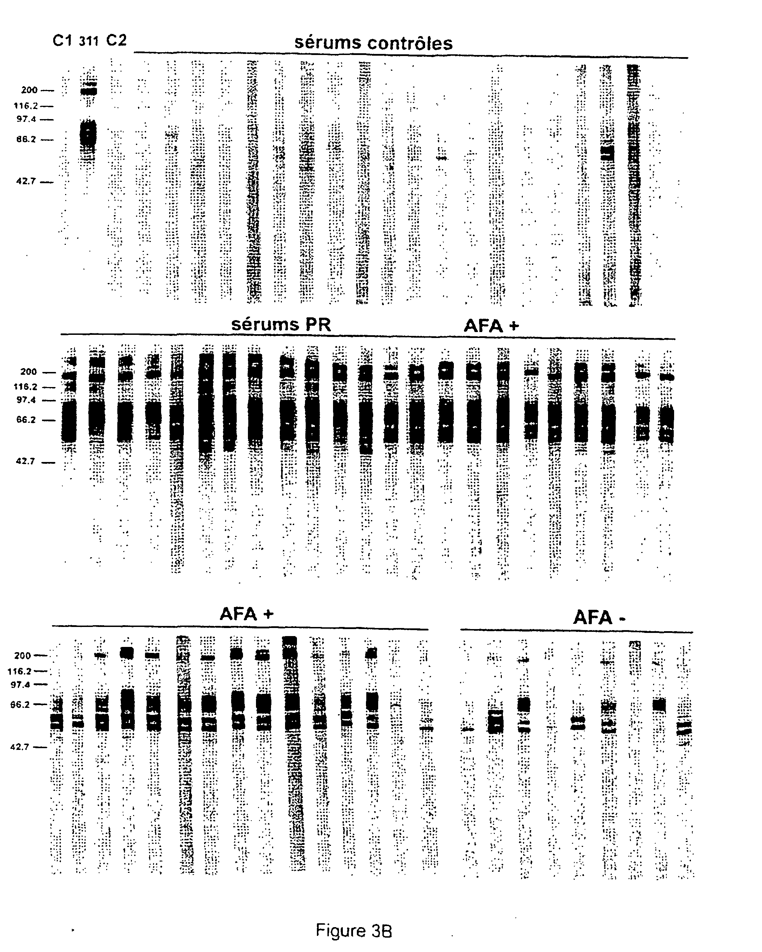

Les résultats sont illustrés par la Figure 3A dans le cas du fibrinogène non-déiminé, et par la Figure 3B dans le cas du fibrinogène déiminé.

### Légende de la Figure 3 :

Figure 3 A : fibrinogène humain purifié non déiminé ;
   - 311 = anticorps monoclonal anti-fibrinogène 311 ;
   - sérums contrôles = sérums issus de patients atteints d'autres rhumatismes inflammatoires que la PR ou de donneurs sains ;
   - sérums PR = sérums rhumatoïdes ;
      * AFA+ = AFA-positifs ;
      * AFA- = AFA-négatifs ;
Figure 3 B : fibrinogène humain purifié déiminé par une PAD ;
   - 311 = anticorps monoclonal anti-fibrinogène 311 ;
   - C1 = anticorps de mouton dirigés contre les IgG de souris ;
   - C2 = anticorps de mouton dirigés contre la protéine-A;
   - sérums contrôles = sérums issus de patients atteints d'autres rhumatismes inflammatoires que la PR ou de donneurs sains ;
   - sérums PR = sérums rhumatoïdes :
      * AFA+ = AFA-positifs ;
      * AFA- = AFA-négatifs.

### Fibrinogène non-déiminé

Après séparation en PAGE-SDS, dans les conditions décrites dans l'exemple 1 ci-dessus, le fibrinogène non déiminé est composé de 3 polypeptides de poids moléculaires apparents respectifs de 48 kDa, 58 kDa et 69 kDa, correspondant aux masses moléculaires apparentes attendues des chaînes polypeptidiques α, β et γ composant la protéine (résultats non présentés). L'anticorps monoclonal anti-fibrinogène "311" reconnaît fortement les chaînes polypeptidiques α et β et t=ès faiblement la chaîne polypeptidique γ (Figure 3A).

Des antisérums spécifiques de chacune des chaînes α, β and γ du fibrinogène ont aussi montré une réactivité vis-à-vis de la chaîne contre laquelle ils étaient respectivement dirigés (résultats non illustrés).

### Déimination du fibrinogène

Une peptidyl-arginine déiminase (PAD) purifiée à partir de muscle squelettique de lapin (Sigma, St Louis, MO) a été utilisée. Le fibrinogène humain (Calbiochem, San Diego, CA) a été incubé à la concentration de 0,86 mg/ml, en présence ou en absence de PAD (7 U/mg de protéine) pendant 2h à 50°C, en tampon Tris 0,1 M, HCl pH 7,4, contenant 10 mM de CaCl2, et 5 mM de DTT. Ces conditions sont celles qui ont préalablement permis de générer les épitopes reconnus par les AAF sur une filaggrine recombinante humaine [GIRBAL-NEUHAUSER et al., J. Immunol . , 162, 585-594, (1999)] . La déimination a ensuite été arrêtée par addition de SDS à 2 % et chauffage à 100°C pendant 3 mn.

Après une déimination de 2 heures, la mobilité électrophorétique en SDS-PAGE des deux polypeptides α et β s'est modifiée, celle du polypeptide γ est restée inchangée. En effet, la protéine correspondant à la chaîne α est alors apparue sous la forme d'une bande diffuse de 82 à 95 kDa et a été immunodétectée à la fois par l'anticorps monoclonal anti-fibrinogène "311" (Figure 3B) et par l'antisérum dirigé contre la chaîne α du fibrinogène (résultats non illustrés).

La protéine correspondant à la chaîne β est apparue sous la forme d'un doublet bien défini de poids moléculaire 58 kD pour la bande inférieure et 60 kD pour la bande supérieure, qui n'a pas été reconnu par l'anticorps monoclonal anti-fibrinogène "311" (Figure 3B) mais a été immunodétecté par l'antisérum de lapin dirigé contre la chaîne β recombinante du fibrinogène humain (résultats non illustrés).

Aucune réactivité de la chaîne α ou de la chaîne β n'est observée avec les anticorps C1 et C2.

### Réactivité des sérums

La réactivité des sérums vis-à-vis des chaînes α et β du fibrinogène non déiminé s'est avérée nulle ou très faible et ne concernait que quelques rares sérums n'appartenant à aucun sous-groupe particulier.

En revanche, après déimination, les polypeptides correspondant aux chaînes α et β déiminées sont fortement réactifs avec les AAF purifiés (résultats non-illustrés) et avec la totalité des 37 sérums rhumatoïdes AAF-positifs (à l'exception de celui qui possède le titre le plus faible en AAF) . Par ailleurs, 6 sérums rhumatoïdes AAF-négatifs sur 10 ont aussi clairement reconnu les polypeptides α ou β déiminés : 2 ont immunodétecté le polypeptide α et le doublet polypeptidique β, 3 autres ont seulement immunodétecté le doublet polypeptidique β et 1 seul a immunodétecté exclusivement le polypeptide α. Par contre, à l'exception d'un sérum issu d'un patient atteint d'un syndrome de Sjögren réactif sur le doublet polypeptidique β, aucun des sérums contrôles -n'a immunodétecté le fibrinogène déiminé.

L'affinité des sérums rhumatoïdes AAF-positifs vis-à-vis des deux polypeptides déiminés α et β s'est révélée légèrement variable d'un sérum à l'autre. Ainsi, 6 sérums, alors qu'ils détectaient fortement le polypeptide β, n'ont que très faiblement reconnu le polypeptide α. De même, 3 sérums fortement réactifs vis-à-vis du polypeptide α n'ont pas détecté le polypeptide àéiminé β. Par ailleurs, l'intensité de marquage des deux polypeptides paraît globalement proportionnelle au titre en AAF des sérums. Il est à noter que les sérums réactifs sur les polypeptides α et β du fibrinogène déiminés ont également été réactifs vis-à-vis de polypeptides de haut poids moléculaire (supérieur à 200 kD) générés lors de la déimination du fibrinogène. Ces polypeptides clairement réactifs avec les anticorps anti-fibrinogène sont très probablement des aggrégats de chaînes de fibrinogène.

En conclusion, la reconnaissance des polypeptides α et β du fibrinogène par les sérums rhumatoïdes est non seulement entièrement dépendante de leur déimination, puisque les polypeptides non-déiminés ne sont jamais reconnus, mais elle est également clairement liée à la réactivité antifilaggrine de ces sérums. Il est à noter que ces polypeptides déiminés permettent de détecter avec une grande sensibilité les AAF présents dans les sérums rhumatoïdes.

Ces résultats démontrent clairement que les cibles antigéniques des AAF dans les articulations synoviales rhumatoïdes sont des formes déiminées de la chaîne α et de la chaîne β de la fibrine humaine.

## Revendications

1. Polypeptide citrulliné reconnu par des autoanticorps anti-filaggrine spécifiques de la polyarthrite rhumatoïde, **caractérisé en ce qu'**il est dérivé de tout ou partie de la séquence de la chaîne α ou de la chaîne β d'une fibrine de vertébré par substitution d'au moins un résidu arginine par un résidu citrulline.

2. Polypeptide citrulliné selon la revendication 1, dérivé d'une séquence d'au moins 5 acides aminés consécutifs de la chaîne α ou de la chaîne β d'une fibrine de vertébré.

3. Polypeptide citrulliné selon une quelconque des revendications 1 ou 2, **caractérisé en ce que** ladite fibrine de vertébré est une fibrine de mammifère, de préférence humaine.

4. Utilisation d'un polypeptide selon une quelconque des revendications 1 à 3 pour le diagnostic *in vitro* de la polyarthrite rhumatoïde.

5. Composition antigénique pour le diagnostic de la présence d'auto-anticorps spécifiques de la polyarthrite rhumatoïde dans un échantillon biologique, **caractérisée en ce qu'**elle contient au moins un polypeptide citrulliné selon une quelconque des revendications 1 à 3, éventuellement marqué et/ou conjugué avec une molécule porteuse.

6. Procédé de détection des auto-anticorps spécifiques de la polyarthrite rhumatoïde dans un échantillon biologique, lequel procédé est **caractérisé en ce qu'**il comprend :
- la mise en contact dudit échantillon biologique avec au moins un polypeptide selon une quelconque des revendications 1 à 3, dans des conditions permettant la formation d'un complexe antigène/anticorps avec les auto-anticorps spécifiques de la polyarthrite rhumatoïde éventuellement présents ;
- la détection, par tous moyens appropriés, du complexe antigène/anticorps éventuellement formé.

7. Nécessaire pour la détection des auto-anticorps spécifiques de la polyarthrite rhumatoïde dans un échantillon biologique, **caractérisé en ce qu'**il comprend au moins un polypeptide selon une quelconque des revendications 1 à 3, ainsi que des tampons et réactifs appropriés pour la constitution d'un milieu réactionnel permettant la formation d'un complexe antigène/anticorps, et/ou des moyens de détection dudit complexe antigène/anticorps.

8. Utilisation d'un polypeptide citrulliné selon une quelconque des revendications 1 à 3 pour l'obtention d'un médicament.

9. Utilisation selon la revendication 8, **caractérisée en ce que** ledit médicament est destiné à neutraliser la réponse auto-immune associée à la PR.

10. Composition pharmaceutique, **caractérisée en ce qu'**elle contient en tant que principe actif, au moins un polypeptide citrulliné selon une quelconque des revendications 1 à 3.

## Patentansprüche

1. Citrullinhaltiges Polypeptid, das von für rheumatoide Polyarthritis spezifischen Anti-Filaggrin-Autoantikörpern erkannt wird, **dadurch gekennzeichnet, dass** es ganz oder teilweise von der Sequenz der α- oder der ß-Kette eines Vertebraten-Fibrins durch Substitution von mindestens einem Argininrest durch einen Citrullinrest abgeleitet ist.

2. Citrullinhaltiges Polypeptid nach Anspruch 1, das von einer Sequenz von mindestens 5 aufeinanderfolgenden Aminosäuren der α- oder der ß-Kette eines Vertebraten-Fibrins abgeleitet ist.

3. Citrullinhaltiges Polypeptid nach einem der Ansprüche 1 oder 2, **dadurch gekennzeichnet, dass** das Vertebraten-Fibrin ein Säuger-Fibrin, vorzugsweise menschliches Fibrin, ist.

4. Verwendung eines Polypeptids nach einem der Ansprüche 1 bis 3 zur In-vitro-Diagnostik von rheumatoider Polyarthritis.

5. Antigene Zusammensetzung zur Diagnostik des Vorhandenseins für rheumatoide Polyarthritis spezifischer Autoantikörper in einer biologischen Probe, **dadurch gekennzeichnet, dass** sie mindestens ein citrullinhaltiges Polypeptid nach einem der Ansprüche 1 bis 3, das gegebenenfalls markiert und/oder mit einem Trägermolekül konjugiert ist, enthält.

6. Verfahren zum Nachweis von für rheumatoide Polyarthritis spezifischen Autoantikörpern in einer biologischen Probe, wobei das Verfahren **dadurch gekennzeichnet ist, dass** es Folgendes umfasst:
- In-Kontakt-bringen der biologischen Probe mit mindestens einem Polypeptid nach einem der Ansprüche 1 bis 3 unter Bedingungen, welche die Bildung eines Antigen/Antikörper-Komplexes mit den gegebenenfalls vorhandenen, für rheumatoide Polyarthritis spezifischen Autoantikörpern erlauben;
- den Nachweis des gegebenenfalls gebildeten Antigen/Antikörper-Komplexes durch jedes geeignete Mittel.

7. Kit zum Nachweis von für rheumatoide Polyarthritis spezifischen Autoantikörpern in einer biologischen Probe, **dadurch gekennzeichnet, dass** es mindestens ein Polypeptid nach einem der Ansprüche 1 bis 3 sowie zur Herstellung eines Reaktionsmediums, das die Bildung eines Antigen/Antikörper-Komplexes gestattet, geeignete Puffer und Reagenzien und/oder Mittel zum Nachweis des Antigen/Antikörper-Komplexes umfasst.

8. Verwendung eines citrullinhaltigen Polypeptids nach einem der Ansprüche 1 bis 3 zur Gewinnung eines Medikaments.

9. Verwendung nach Anspruch 8, **dadurch gekennzeichnet, dass** das Medikament zur Neutralisation der Autoimmunantwort in Verbindung mit rheumatoider Polyarthritis bestimmt ist.

10. Pharmazeutische Zusammensetzung, **dadurch gekennzeichnet, dass** sie als Wirkstoff mindestens ein citrullinhaltiges Polypeptid nach einem der Ansprüche 1 bis 3 umfasst.

## Claims

1. Citrullinated polypeptide recognised by rheumatoid-polyarthritis-specific anti-filaggrin autoantibodies, **characterised in that** it is derived from all or part of the sequence of the α chain or the β chain of a vertebrate fibrin by substitution of at least an arginine residue by a citrulline residue.

2. Citrullinated polypeptide according to claim 1, derived from a sequence of at least 5 consecutive amino acids of the α chain or the β chain of a vertebrate fibrin.

3. Citrullinated polypeptide according to either claim 1 or claim 2, **characterised in that** the vertebrate fibrin is a mammalian fibrin, preferably a human fibrin.

4. Use of a polypeptide according to any one of claims 1 to 3 for in vitro diagnosis of rheumatoid polyarthritis.

5. Antigen composition for diagnosing the presence of rheumatoid-polyarthritis-specific autoantibodies in a biological sample, **characterised in that** it contains at least a citrullinated polypeptide according to any one of claims 1 to 3, optionally marked with and/or joined to a carrier molecule.

6. Process for detecting rheumatoid-polyarthritis-specific autoantibodies in a biological sample, which process is **characterised in that** it comprises:
- bringing into contact the biological sample with at least a polypeptide according to any one of claims 1 to 3, under conditions allowing the formation of an antigen/antibody complex with the rheumatoid-polyarthritis-specific autoantibodies which may be present;
- detecting, by any appropriate means, the antigen/antibody complex which may have been formed.

7. Kit for detecting rheumatoid-polyarthritis-specific autoantibodies in a biological sample, **characterised in that** it comprises at least a polypeptide according to any one of claims 1 to 3, and suitable buffers and reagents for constituting a reaction medium allowing the formation of an antigen/antibody complex, and/or means for detecting the antigen/antibody complex.

8. Use of a citrullinated polypeptide according to any one of claims 1 to 3 for obtaining a medicament.

9. Use according to claim 8, **characterised in that** the medicament is intended to neutralise the auto-immune response which is associated with rheumatoid polyarthritis.

10. Pharmaceutical composition, **characterised in that** it contains, as the active ingredient, at least a citrullinated polypeptide according to any one of claims 1 to 3.
